# EUROPEAN PATENT APPLICATION

(11) **EP 2 949 312 A1**
(43) Date of publication of application: **02.12.2015**
(21) Application number: 15169964.2
(22) Date of filing: 29.05.2015
(51) Int. Cl.: A61K 8/26, A61K 8/31, A61K 8/37, A61K 8/39, A61K 8/41, A61Q 15/00, A61K 8/73, A61K 8/92, A61K 8/04

(54) **AEROSOL DEODORANT ANTIPERSPIRANT COMPOSITIONS**

(30) Priority: 30.05.2014 US 201462005016 P; 13.04.2015 US 201514684704
(71) Applicant: Johnson & Johnson Consumer Companies Inc., Skillman, NJ 08558 (US)
(72) Inventor: LEMOS ANCONI, Glasiela, 12245-500 Sao Jose dos Campos - SP (BR); DE CASTRO MONTEIRO LOFFREDO, Luciana, 12242-431 Sao Jose dos Campos - SP (BR); PASSERO, Alan, 12305-650 Jacarei (BR)
(74) Representative: Carpmaels & Ransford LLP

(57) **Abstract**

The present invention relates to a deodorant antiperspirant composition including: an anti-perspirant; a deodorant; an oil absorbent selected from the group consisting of aluminum starch octenylsuccinate, tapioca starch, and polymethylsilsesquioxane; a carrier comprising dodecane, an ester selected from the group consisting of isopropyl myristate, ethylhexyl stearate, isocetyl stearate, isopropyl isostearate, and myristyl myristate, and helianthus annuus seed oil; a suspending agent; and at least one propellant, wherein the composition is substantially free of silicone.

## Description

### Field of the Invention

The present invention relates to silicone-free, aerosol deodorant antiperspirant compositions. The compositions are effective for 72 hours of deodorant protection and 24 hours of antiperspirant protection.

### Background of the Invention

The present invention relates to deodorant antiperspirant compositions that can be dispensed by aerosol. Known aerosol deodorant antiperspirant compositions are applied in a liquid form and typically contain silicone containing ingredients such as dimethicone, dimethicol and cyclomethicone. There are various side effects associated with the different silicones. For example, dimethicone may cause mild itching, burning or stinging. Other side effects may be more severe; these side effects may also include allergic reactions such as rash, hives, difficulty with breathing, tightness in the chest, swelling of the mouth, face, lips or tongue, severe or persistent itching, burning, stinging or worsening dryness.

Published patent application WO03/041674 discloses a roll-on formulation of the water-in-oil type. Water-in oil formulations are known to the skilled formulator to present different problems from oil-in-water cosmetic formulations; for example, they act differently because the external phase is different. Such compositions are likely to have problems of slow drying time. United States patent applications US20090220444, US20090214457, US20090123398, and US20100143426 teach silicone free antiperspirant compositions.

There is a need for an aerosol deodorant antiperspirant that dries quickly and is aesthetically pleasing, but does not contain silicone.

### Summary of the Invention

The present invention provides a deodorant antiperspirant aerosol composition including: an anti-perspirant; a deodorant; an oil absorbent selected from the group consisting of aluminum starch octenylsuccinate, tapioca starch, and polymethylsilsesquioxane; a carrier comprising dodecane, an ester selected from the group consisting of isopropyl myristate, ethylhexyl stearate, isocetyl stearate, isopropyl isostearate, and myristyl myristate, and helianthus annuus seed oil; a suspending agent; and at least one propellant, wherein the composition is substantially free of silicone.

Compositions according to the invention provide fast drying and long lasting efficacy. The compositions leave a dry touch on the skin and the antiperspirant and deodorant benefits may last up to 72 hours.

### Detailed Description of the Invention

The compositions of the invention are substantially free of silicone. As used herein, "substantially free of silicone" means containing less than 0.1%, preferably less than 0.01% percent by weight silicone. In one embodiment, the compositions are completely free of silicones, i.e., contain 0% by weight silicone.

The instant invention utilizes a concentration of an antiperspirant active effective to reduce or control sweating or reduce or eliminate body malodour. The amount of antiperspirant may range from about 0.1% to about 15%, or from about 5% to about 10%, or from about 4% to about 8% by weight, based on the total weight of the composition.

The antiperspirant active is typically an astringent aluminium and/or zirconium salt, including astringent inorganic salts, astringent salts with organic anions and complexes of such salts. Preferred astringent salts include aluminium, zirconium and aluminium/zirconium halides and halohydrate salts, such as especially chlorohydrates. Activated chlorohydrates can be incorporated, if desired. Some literature employs alternative terminology for chlorohydrates, such as basic aluminium chloride, and aluminium chlorhydrex.

Aluminium halohydrates are usually defined by the general formula Al₂(OH)ₓQ_{y}. wH₂O in which Q represents respectively chlorine, bromine or iodine, (and especially chlorine to form a chlorohydrate) x is variable from 2 to 5 and x + y = 6 while wH₂O represents a variable amount of hydration.

Zirconium actives can usually be represented by the empirical general formula: ZrO(OH)_{2n-nz}B_{z} · wH₂O in which z is a variable in the range of from 0. 9 to 2.0 so that the value 2n-nz is zero or positive, n is the valency of B and B is selected from the group consisting of chlorine (to form a chlorohydrate), other halide, sulphamate, sulphate and mixtures thereof. Possible hydration to a variable extent is represented by wH₂O. Preferably, B represents chlorine and the variable z lies in the range from 1.5 to 1.87. In practice, such zirconium salts are usually not employed by themselves, but as a component of a combined aluminium and zirconium-based antiperspirant.

The above aluminium and zirconium salts may have co-ordinated and/or bound water in various quantities and/or may be present as polymeric species, mixtures or complexes. In particular, zirconium hydroxy salts often represent a range of salts having various amounts of the hydroxy group. Zirconium aluminium chlorohydrate may be particularly preferred.

Antiperspirant complexes based on the above-mentioned astringent aluminium and/or zirconium salts can be employed. The complex often employs a compound with a carboxylate group, and advantageously this is an amino acid. Examples of suitable amino acids include dl-tryptophan, dl-phenylalanine, dl-valine, dl-methionine and 3-alanine, and preferably glycine which has the formula CH₂(NH₂)COOH.

In some compositions, it is highly desirable to employ complexes of a combination of aluminium chlorohydrates and zirconium chlorohydrates together with amino acids such as glycine, which are disclosed in US 3792063 (Luedders et al). Certain of those Al/Zr complexes are commonly called ZAG in the literature. ZAG actives generally contain aluminium, zirconium and chloride with an Al/Zr ratio in a range from 2 to 10, especially 2 to 6, an Al/Cl ratio from 2.1 to 0.9 and a variable amount of glycine. Actives of this preferred type are available from Westwood, from Summit and from Reheis.

Compositions according to the present invention further include a deodorant active. The deodorant active can be selected from any deodorant active known in the cosmetic art such as antimicrobial actives such as polyhexamethylene biguanides, e.g., those available under the trade name COSMOCIL, or chlorinated aromatics, e.g., triclosan available under the trade name IRGASAN, non-microbiocidal deodorant actives such as triethylcitrate, bactericides and bacteriostats. Yet other deodorant actives can include bactericidal zinc salts such as zinc ricinoleate. The concentration of such alternative deodorant active is desirably from 0.01 to 5% and in many instances is from 0.1 to 1% by weight of the composition.

Compositions according to the present invention may include polyglyceryl-3 caprylate, triclosan, farnesol, trichlorocarban or combinations thereof which may function as an emollient and a deodorant by killing bacteria. TEGO® Cosmo P 813 from Evonik is a mild vegetable based cosmetic coemulsifier that may also be useful in this context. TEGO® Cosmo P 813 has anti-microbial properties. It reliably reduces odor-causing bacteria on the skin at very low concentrations. TEGO® Cosmo P 813 is an ester, with lipophylic character, which means it is an emollient to skin, an important benefit to sensitive underarm skin. It is also advantageous for body care, because while other anti-microbials have constant activity on the skin's surface, this active is activated by the surface flora associated with the sweating process. TEGO® Cosmo P 813 is cleaved by bacterial lipases, releasing caprylic acid, which functions as the antimicrobial agent.

Compositions according to the invention contain an oil absorbent selected from the group consisting of aluminum starch octenylsuccinate (also known as modified corn starch), tapioca starch, and polymethylsilsesquioxane. The amount of oil absorbent may range from about 0.001% to about 3% by weight, for example from about 0.01% to about 0.5% based on the total weight of the composition.

Compositions according to the present invention include a liquid carrier comprising isododecane, an ester selected from the group consisting of isopropyl myristate, ethylhexyl stearate, isocetyl stearate, isopropyl isostearate, and myristyl myristate, and helianthus annuus seed oil. This combination of liquids delivers desirable aesthetics and allows for effective application to the skin of the anti-perspirant.

Isododecane is a clear, colourless and odourless, volatile liquid, which makes it suitable for use in colour cosmetics like mascara, eyeliner, lip products, antiperspirant or any product where improved wear properties and no residues are wanted. It does not leave an oily residue. Isododecane is a volatile, lipophilic component for deodorant sprays and hair care applications. It is a hydrocarbon ingredient used as a solvent. Isododecane enhances the spreadability of products and has a weightless feel on skin. It may be used in the compositions of the present invention at from about 0.10% to about 20% by weight, for example from about 1% to about 5% by weight based on the total weight of the composition.

Isopropyl myristate is a synthetic oil used as an emollient, thickening agent, or lubricant in beauty products. Isopropyl myristate is a popular cosmetic and pharmaceutical ingredient. It is most often used an additive in aftershaves, shampoos, bath oils, antiperspirants, deodorants, oral hygiene products, and various creams and lotions. A unique characteristic of isopropyl myristate is its ability to reduce the greasy feel caused by the high oil content of other ingredients in a product. This synthetic oil is often added to beauty products to give them a slicker, sheer feel rather than an oily one. There are many esters similar in function to this chemical, including ethylhexyl stearate, isocetyl stearate, isopropyl isostearate, and myristyl myristate, which may also be used alone or in combination with each other or isopropyl myristate. Isopropyl myristate is easily absorbed by the skin, ensuring quick penetration of a formula's ingredients. It may be used in the compositions of the present invention at from about 0.10% to about 20% by weight, for example from about 1% to about 10% by weight based on the total weight of the composition.

Helianthus annuus seed oil is a high oleic sunflower oil offering functional advantages in many applications. The fatty acid composition of helianthus annuus seed oil is radically different to conventional sunflower oil. Derived from selected varieties of sunflower seeds which have been developed using conventional plant breeding techniques, helianthus annuus seed oil is high in monounsaturated fats and low in saturates. It may be used in the compositions of the present invention at from about 0.01% to about 3% by weight, for example from about 0.1% to about 1% by weight based on the total weight of the composition.

Aerosol antiperspirants and deodorants are designed to work via a thin film which is propelled onto the skin. To create this film, products contain low, medium and high pressure propellants which produce a strong, but comfortable, spray to reach the skin. Propellants utilized in the compositions of the present invention include butane, isobutane, propane and combinations thereof. Propellants may be used in the compositions of the present invention at from about 30% to about 95% by weight, for example from about 70% to about 90% by weight based on the total weight of the composition. The ratio of butane and/or isobutane to propane may range from 80:20 to 60:40.

Compositions according to the present invention also include a suspending agent. For example, the suspending agent may be stearalkonium hectorite, which is designed to impart rheological control and suspension and is a suitable thickener for compositions of the present invention. It is a highly efficient rheological additive for intermediate to high polarity systems such as cyclomethicones, esters, triglycerides, vegetable oils, alcohols and ketones. It may be used in the compositions of the present invention at from about 0.01% to about 5% by weight, for example from about 0.1% to about 2% by weight based on the total weight of the composition.

Fragrance may be included in the final product. The amount of fragrance may be from about 0.1% to about 3% by weight, or from about 0.3% to about 2% by weight, based on the total weight of the composition.

Compositions according to the present invention demonstrate a particularly desirable combination of product attributes such as improved speed of drying, superior greasiness and avoidance of excessive stickiness on application.

Preferably, the composition is in the form or an emulsion. Such emulsion is made by first preparing separate aqueous and oil mixtures which are brought together before shearing. The temperature of the respective phases can be raised, where necessary, to accelerate dissolution of the emulsifier, for example to above 50°C.

### Examples

### Example 1

Preliminary formulation tests indicated that isopropyl myristate alone would not suffice as the carrier because of staining issues. The samples also had an oily feel. The combination of isopropyl myristate with isododecane and helianthus annuus seed oil provided the desired attributes.

Using the ingredients in Table 1 below and the procedure below, a bulk composition (no propellant) according to the invention was prepared. The final formulation would further include a propellant composition (thereby decreasing the amount of each ingredient based on the total weight of the composition).

**Table 1**

| INGREDIENT | % w/w |
|---|---|
| A. Aluminum Chlorohydrate | 40.00 |
| B. Isopropyl Myristate | 35.00 |
| C. Isododecane | 20.47 |
| D. Stearalkonium Hectorite | 2.00 |
| E. Helianthus Annuus Seed Oil | 1.00 |
| F. Polyglyceryl-3 Caprylate | 0.50 |
| G. Propylene Carbonate | 0.67 |
| H. Butylated hydroxytoluene | 0.15 |
| I. Aluminum Starch Octenylsuccinate | 0.10 |
| J. Tocopheryl Acetate | 0.10 |
| K. Glycerin; Water; PEG-40 Hydrogenated Castor Oil; Trideceth-9; Nonfat Dry Milk; Gossypium Herbaceum (Cotton) Seed Oil; 1,2-Hexanediol; Caprylyl Glycol; Disodium EDTA; Citric Acid; Sodium Hydroxide | 0.01 |

### Procedure

1. Pre-mix: Stearalkonium Hectorite Gel - Weigh B and D in an auxiliary tank and mix for 15 minutes, after that homogenize for 15 minutes. Add G and mix for 15 minutes, then pass through homogenizer for 15 min.
2. Powder in oil suspension - Add C and H in a main tank and stir until H is completely solubilized. Add Stearalkonium Hectorite Gel (Pre-mix). Add E, F, J and K to main tank and stir. Add A and I under mixing.

### Example 2

Using the ingredients in Table 2 below and the procedure below, an aerosol spray-on composition according to the invention was prepared.

**Table 2**

| INGREDIENT | % w/w |
|---|---|
| A. Aluminum Chlorohydrate | 6.00 |
| B. Isopropyl Myristate | 5.25 |
| C. Isododecane | 3.07 |
| D. Stearalkonium Hectorite | 0.30 |
| E. Helianthus Annuus Seed Oil | 0.15 |
| F. Polyglyceryl-3 Caprylate | 0.075 |
| G. Propylene Carbonate | 0.10 |
| H. ButilHidroxiTolueno | 0.023 |
| I. Aluminum Starch Octenylsuccinate | 0.015 |
| J. Tocopheryl Acetate | 0.015 |
| K. Glycerin; Water; PEG-40 Hydrogenated Castor Oil; Trideceth-9; Nonfat Dry Milk; Gossypium Herbaceum (Cotton) Seed Oil; 1,2-Hexanediol; Caprylyl Glycol; Disodium EDTA; Citric Acid;Sodium Hydroxide | 0.002 |
| L. Butane; Propane | 85,00 |

### Procedure

1. Pre-mix: Stearalkonium Hectorite Gel - Weigh B and D in an auxiliary tank and mix for 15 minutes, then homogenize for 15 minutes. Add G and mix for 15 minutes, then pass through homogenizer for 15 minutes.
2. Powder in oil suspension - Add C and H in a main tank and stir until H is completely solubilized. Add Stearalkonium Hectorite Gel (Pre-mix). Add E, F, J and K to main tank and stir. Finally add A and I under mixing to produce a concentrate.
3. Aerosol Filling - Fill the concentrate into an aluminum can with Butane/Propane propellant. Concentrate/Propellant proportion is 15:85.

### Example 3

The composition of Example 1 was compared with four commercially available anti-perspirant products containing the following ingredients:

**Table 3**

| **GARNIER Bio Mineral** | **DOVE Original** | **NIVEA Dry Comfort** | **REXONA Powder** |
|---|---|---|---|
| isobutane, butane, propane, dimethicone, aluminum chlorohydrate, triethyl citrate, parfum (fragrance), isopropyl palmitate, stearalkonium bentonite, perlite, dimethiconol, limonene, hexyl cinnamal, talc, benzyl salicylate, linalool, butylphenyl methylpropional, citronellol, geraniol, alpha-isomethyl ionone, citral | butane, isobutane, propane, aluminium chlorohydrate, PPG-14 butyl ether, cyclomethicone, parfum, disteardimonium hectorite, helianthus annuus seed oil, C12-15 alkyl benzoate, octyldodecanol, BHT, dimethiconol, propylene carbonate, tocopheryl acetate, alpha-isomethyl ionone, benzyl alcohol, benzyl salicylate, butylphenyl methylpropional, citronellol, coumarin, eugenol, geraniol, hexyl cinnamal, linalool | butane, isobutane, propane, cyclomethicone, aluminum chlorohydrate, parfum, disteardimonium hectorite, dimethicone, octyldodecanol, butyloctanoic acid, persea gratissima oil (avocado), tocopheryl acetate, magnesium aluminum silicate, d-limonene, geraniol, citronellol | butane, isobutane, propane, aluminium chlorohydrate, PPG-14 butyl ether, cyclomethicone, parfum, disteardimonium hectorite, caprylic/capric triglyceride, hydrated silica, gelatin crosspolymer, aqua, cellulose gum, sodium benzoate, sodium starch octenylsuccinate, maltodextrin, hydrolyzed corn starch, silica, alpha-isomethyl ionone, benzyl salicylate, butylphenyl methylpropional, cinnamyl alcohol, citronellol, coumarin, geraniol, hexyl cinnamal, limonene, linalool |

Product drying time test - The product drying of each of the five compositions was determined by a thermo gravimetric technique, which consists basically of a thermal analysis module. The module used was the Thermogravimetric Analyzer (TGA) Model 2950 from TA Instruments. The TGA consisted of a microbalance, a chamber (oven) and a controller (computer interface). The TGA measures the weight of a sample as a function of temperature and/or time under controlled atmosphere. In this specific case, the controlled atmosphere not only related to the type of the gas but also to the level of relative humidity inside of the chamber. The TGA analysis conditions were as follows:
1. Adjust the purge gas as follows: Synthetic air - 150 mL/min of humid air and 0 mL/min of dry air.
2. Evenly spread 2.0 mg of the product on the sample holder.
3. Starting from 25°C heat at a ramp of 5°C/min up to 36°C.
4. Keep the conditions isothermal for 60 minutes.
5. The relative humidity is kept constant at 50%.

The results (average of 3 replicates) are shown in Table 4 below.

**Table 4**

| Sample | Drying Ratio | Loss of half weight (minutes) |
|---|---|---|
| Example 1 Composition | 1.62 | 5.65 |
| DOVE | 1.80 | 20.17 |
| GARNIER | 1.47 | 22.23 |
| NIVEA | 2.18 | 39.40 |
| REXONA | 1.80 | 21.12 |

The composition of Example 1 performed statistically similarly to the DOVE and REXONA products. Statistical difference was observed between the NIVEA and GARNIER products. For the parameter "Loss of half weight", which refers to the time taken for a sample to reduce to 50% of its initial weight, the composition of Example 1 provided statistically better performance in comparison to all the commercially available products. The composition according to the invention provided a fast drying velocity.

Oily residue test - Oily materials are mainly composed of non-polar molecules. Non-polar materials have affinity for non-polar surfaces, and the opposite is true: polar molecules, such as water, do not have affinity for non-polar substrates. This is the basic principle of the methodology here employed.

The weight of oil remaining on a thin, non-polar, porous, plastic substrate after application of the Example 1 composition and the four comparative compositions was measured. This modeled the level of undesirable greasy feeling on the skin after product application. The higher the amount of oily material on the substrate after drying, or the lesser its absorption by the skin, the larger the amount of oily material "captured" by the substrate, and consequently, the higher the undesired greasy feeling resulting from the product use. The test was performed as follows under acclimated room conditions, temperature (22±1)°C, relative humidity (55±2)%, and atmospheric pressure.

The plastic film from JOHNSON & JOHNSON CLEAN & CLEAR Oil Absorbing Sheets was used to make the substrates. The JOHNSON & JOHNSON CLEAN & CLEAR Oil Absorbing Sheets comprise a polypropylene film treated with mineral oil, dimethylbenzylidene sorbitol and ultramarines, which is a mineral-derived blue pigment composed of sodium, aluminum, silicate and sulfate. The absorbing sheets were cut into discs of 3.2cm diameters (8cm² area). Next, a 5cm² circular area was drawn on a human subject's inner forearm using a surgical marker. Each product was applied to at least 4 subjects. 10mg of a given test product was applied to the circular test area, obtaining a uniform film and allowed to dry for three minutes. Next a disc, previously weighed, was placed over the treated area. A cylindrical weight of 70g having a base diameter 2.8cm (6.2cm² area) was placed on the central region of the disc for 30sec. This ensured a standardized and constant pressure of 11.4g/cm². The disc was removed immediately after the 30 seconds and weighed. Weighing accuracy was 0.05mg.

The results (average of 3 replicates) are shown in Table 5 below.

**Table 5**

| Sample | Weight oil on disc after drying (mg) |
|---|---|
| Example 1 Composition | 1.26 |
| DOVE | 1.65 |
| GARNIER | 6.18 |
| NIVEA | 2.18 |
| REXONA | 1.70 |

The composition of Example 1 provided the lowest free oily residue amount on the substrate after application; however this was not statistically different from that performance of the REXONA and DOVE products. The NIVEA and GARNIER products left statistically higher amounts of free oily residue on the substrate.

## Claims

1. A deodorant antiperspirant composition comprising:
an anti-perspirant;
a deodorant;
an oil absorbent selected from the group consisting of aluminum starch octenylsuccinate, tapioca starch, and polymethylsilsesquioxane;
a carrier comprising dodecane, an ester selected from the group consisting of isopropyl myristate, ethylhexyl stearate, isocetyl stearate, isopropyl isostearate, and myristyl myristate, and helianthus annuus seed oil;
a suspending agent; and
at least one propellant, wherein the composition is substantially free of silicone.
